# EUROPÄISCHE PATENTSCHRIFT

(11) **EP 0 863 137 B1**
(45) Veröffentlichungstag und Bekanntmachung des Hinweises auf die Patenterteilung: **24.11.2004**
(21) Anmeldenummer: 98103807.8
(22) Anmeldetag: 04.03.1998
(51) Int. Cl.: C07D 211/58

(54) **Verfahren zur Herstellung von 4-Amino-2,2,6,6-tetramethyl-piperidin**
Process for the preparation of 4-amino-2,2,6,6-tetramethyl-piperidine
Procédé pour la préparation de 4-amino-2,2,6,6-tétraméthyl-pipéridine

(30) Priorität: 06.03.1997 DE 19709260
(43) Veröffentlichungstag der Anmeldung: 09.09.1998
(73) Patentinhaber: BASF AKTIENGESELLSCHAFT, 67056 Ludwigshafen (DE)
(72) Erfinder: Voit, Guido, Dr., 69198 Schriesheim (DE); Witzel, Tom, Dr., 67069 Ludwigshafen (DE); Julius, Manfred, Dr., 67117 Limburgerhof (DE)
(74) Vertreter: Isenbruck, Günter, Dr.

(56) Entgegenhaltungen:
- EP-A- 0 033 529
- EP-A- 0 042 119
- EP-A- 0 449 089
- EP-A- 0 611 137
- EP-A- 0 623 585
- EP-A- 0 714 890
- DE-A- 3 525 387

## Beschreibung

Die vorliegende Erfindung betrifft ein Verfahren zur Herstellung von 4-Amino-2,2,6,6-tetramethyl-piperidin (Triacetondiamin, TAD) aus Triacetonamin (TAA).

TAD ist ein zentrales Zwischenprodukt für die Synthese von Lichtschutzmitteln vom HALS-Typ (HALS = *H*indered *A*mine *L*ight *S*tabilizer), die als Additive für die Stabilisierung von synthetischen Polymeren (z.B. Polyolefinen) eine wichtige Rolle spielen.

EP-B 0 033 529 beschreibt die aminierende Hydrierung von 2,2,6,6-Tetramethyl-piperidin-4-on (Triacetonamin, TAA) zu TAD in Abwesenheit eines Lösungsmittels, bei einem NH₃-Überschuß von 10 bis 50 Mol (pro Mol TAA), einem Druck von 50 bis 500 bar und Temperaturen von 120 bis 220 °C ― also einen einstufigen Prozeß gemäß der nachfolgenden Reaktionsgleichung:

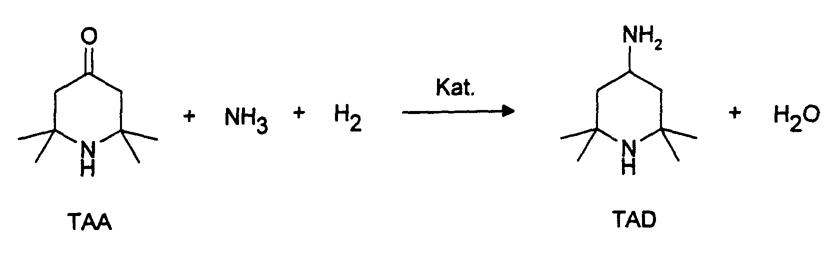

Die EP-B 0 042 119 beschreibt die Synthese von TAD aus TAA, wobei man das Keton TAA zunächst in einem ersten Schritt mit Ammoniak in Gegenwart von anorganischen oder organischen Ionenaustauschern in der Ammoniumform als Iminbildungskatalysatoren unter Bildung des TAA-Imins 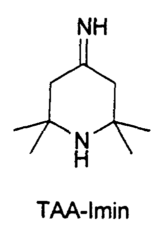 umsetzt; anschließend erfolgt die Hydrierung des TAA-Imins in Gegenwart eines Hydrierkatalysators zum TAD (zweistufiger Prozeß). Ein Nachteil dieses Verfahrens zur TAD-Herstellung liegt darin, daß der dort als Katalysator für den Iminbildungsschritt angewandte Ionenaustauscher teuer und mechanisch bzw. thermisch nur wenig stabil ist.

In der EP-A 0 611 137 wird ein Prozeß zur Herstellung von Aminen aus zyklischen Ketonen beschrieben. Hierbei wird zunächst das Keton (z.B. TAA) mit NH₃ in Gegenwart von Aktivkohle in das entsprechende Imin überführt und dieses anschließend zum Amin (TAD) hydriert (zweistufiger Prozeß).

Die EP-A 0 623 585 beschreibt die aminierende Hydrierung eines zyklischen Ketons (z.B. TAA) oder die Hydrierung eines entsprechenden Imins (TAA-Imin) in Gegenwart eines speziellen Kobaltkatalysators.

Aufgabe der Erfindung ist es, ein Verfahren zur Herstellung von TAD aus TAA bereitzustellen, das unter Kostengesichtspunkten und bezüglich der Art der verwendeten Katalysatoren eine großtechnisch weniger aufwendige Alternative zu den herkömmlichen Verfahren darstellt.

Diese Aufgabe wird durch ein Verfahren zur Herstellung von 4-Amino-2,2,6,6-Tetramethyl-piperidin (TAD) der Formel I 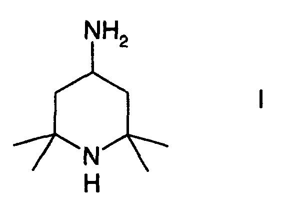 gelöst, wobei in einem ersten Schritt 2,2,6,6-Tetramethyl-piperidin-4-on (TAA) der Formel II

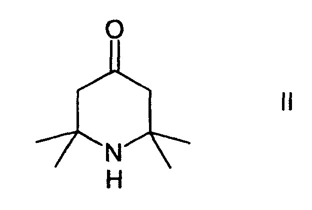

Mit Ammoniak in Gegenwart a) fester saurer Katalysatoren, die im Reaktionsgemisch praktisch unlöslich sind - ausgenommen anorganische und organische Ionenaustauscher in der Ammoniumform-, zum TAA-Imin der Formel III 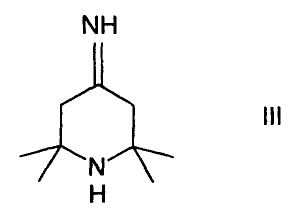 umgesetzt wird und in einem zweiten Schritt das TAA-Imin in Anwesenheit eines Hydrierkatalysators b) hydriert wird.

Außerdem stellt die Erfindung ein Verfahren zur Herstellung von 2,2,6,6-Tetramethylpiperidin-4-imin (TAA-Imin) der Formel III 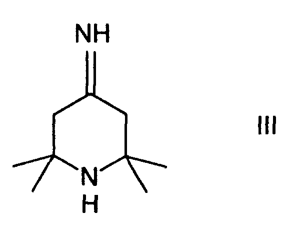 zur Verfügung, wobei 2,2,6,6-Tetramethyl-piperidin-4-on (TAA) der Formel II 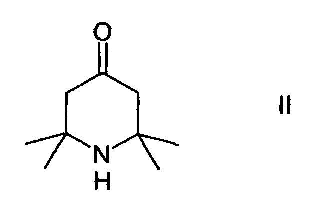 mit Ammoniak in Gegenwart a) fester saurer Katalysatoren, die im Reaktionsgemisch praktisch unlöslich sind - ausgenommen anorganische und organische Ionenaustauscher in der Ammoniumform -, umgesetzt wird.

Es wurde also ein Verfahren gefunden, das über das als Zwischenstufe durch Voriminierung gebildete TAA-Imin verläuft; dieses Verfahren zeichnet sich dadurch aus, dass die Synthese des TAA-Imins in Gegenwart der genannten Katalysatoren durchgeführt wird. Bevorzugte Beispiele für Katalysatoren a) sind Aluminiumhydrosilikate vom Bentonitoder Montmorillonit-Typ oder Aluminiumsilikate von Zeolith-, Mordenit- oder Erionit-Typ.

Das als Zwischenstufe auftretende Imin kann zwar gemäß einer Ausführungsform als Reinsubstanz isoliert werden, bevorzugt ist jedoch bei der Herstellung des TAD die direkt sich anschließende Hydrierung unter üblichen Bedingungen (z.B. 130 C, 250 bar H₂) in Gegenwart des überschüssigen Ammoniaks (bevorzugte Fahrweise). Als Katalysator b) für diesen zweiten Schritt können handelsübliche edelmetallhaltige Hydrierkatalysatoren eingesetzt werden.

Das Verfahren wird bevorzugt in Abwesenheit eines zusätzlichen Lösungsmittels durchgeführt, es können aber gegebenenfalls bezüglich der Reaktanden und Produkte inerte organische Lösungsmittel wie Tetrahydrofuran oder Alkanole (z.B. Methanol) Verwendung finden. Die Umsetzung mit überschüssigem Ammoniak erfolgte insbesondere bei Temperaturen von 20 bis 150°C, bevorzugte Drücke liegen bei 15 bis 500 bar.

Die beiden Reaktionsschritte können diskontinuierlich in Autoklaven oder auch, besonders bevorzugt, kontinuierlich in Rohrreaktoren oder Druckbehälterkaskaden ausgeführt werden.

### Vergleichsbeispiele

### Synthese von 4-Amino-2,2,6,6-Tetramethyl-piperidin (TAD)

### Beispiel 1

In einen vertikalen Druck-Rohrreaktor (Volumen = 50 ml), der mit 37 g Titandioxid (1,5 mm Stränge) gefüllt war, wurde in Sumpffahrweise eine auf 80°C vorgeheizte flüssige Mischung, bestehend aus 40,0 g/h TAA (Reinheit nach GC: 97,3 %) und 792 ml/h Ammoniak, gepumpt. Mittels eines ölbeheizten Doppelmantels wurde die Reaktortemperatur auf 120 °C eingestellt. Der Reaktoraustrag wurde anschließend zusammen mit 43 l/h Wasserstoff von unten nach oben durch einen nachgeschalteten weiteren Rohrreaktor (Volumen = 200 ml), der mit 307 g eines Hydrierkatalysators, mit einem Gehalt an 90 % CoO, 1 % Mn₃O₄, 2 % P₂O₅ und 2 % Na₂O (4 mm Stränge), gefüllt war, bei einem Druck von 250 bar und einer Temperatur von 130 °C gefahren. Der Reaktoraustrag wurde danach in einem Hochdruckabscheider vom überschüssigen Wasserstoff befreit, auf Normaldruck entspannt und überschüssiger Ammoniak abdestilliert. Der Austrag enthielt nach gaschromatographischer Analyse 95 % TAD und 1,9 % Triacetonaminoalkohol (TAA-ol), entsprechend einer TAD-Ausbeute von 98 %

### Beispiel 2

Die Durchführung entsprechend Beispiel 1, mit der Änderung, daß in den ersten Reaktor 41,0 g/h TAA und 1097 ml/h NH₃ gepumpt wurden und in dem zweiten Reaktor 59 l/h Wasserstoff gefahren wurden, lieferte einen Reaktionsaustrag, der 94 % TAD und 1,8 % TAA-ol enthielt. Dies entsprach einer Ausbeute an TAD von 97 %.

### Beispiel 3

Die Durchführung entsprechend Beispiel 1, mit der Änderung, daß in den ersten Reaktor 77,2 g/h TAA und 1217 ml/h NH₃ gepumpt wurden und in dem zweiten Reaktor 58 l/h Wasserstoff gefahren wurden, lieferte einen Reaktionsaustrag, der 92 % TAD und 3,2 % TAA-ol enthielt. Dies entsprach einer Ausbeute an TAD von 95 %.

## Patentansprüche

1. Verfahren zur Herstellung von 4-Amino-2,2,6,6-tetramethyl-piperidin (TAD) der Formel **I**, 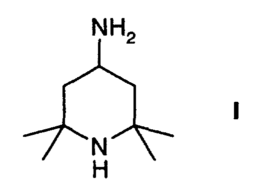 wobei in einem ersten Schritt 2,2,6,6-Tetramethyl-piperidin-4-on (TAA) der der Formel **II** 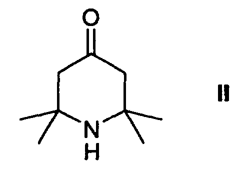 mit Ammoniak in Gegenwart a) fester saurer Katalysatoren, die im Reaktionsgemisch praktisch unlöslich sind, zum TAA-Imin der Formel **III** 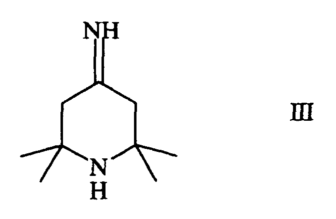 umgesetzt wird,
und
in einem zweiten Schritt das TAA-Imin in Anwesenheit eines Hydrierkatalysators b) hydriert wird,
**dadurch gekennzeichnet, dass** als Katalysator a) Aluminiumhydrosilikate vom Bentonit- oder Montmorillonit-Typ oder Aluminiumsilikate vom Zeolith-, Mordenit- oder Erionit-Typ eingesetzt werden.

2. Verfahren zur Herstellung von 2,2,6,6-Tetramethyl-piperidin-4-imin (TAA-Imin) der Formel **III**, 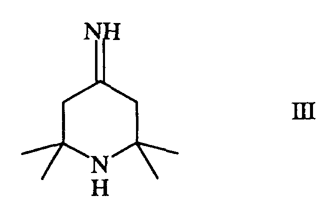 wobei 2,2,6,6-Tetramethyl-piperidin-4-on (TAA) der Formel **II** 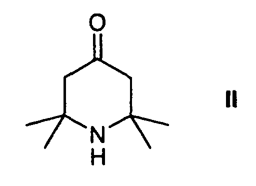 mit Ammoniak in Gegenwart a) fester saurer Katalysatoren, die im Reaktionsgemisch praktisch unlöslich sind, umgesetzt wird, **dadurch gekennzeichnet, dass** als Katalysator a) Aluminiumhydrosilikate vom Bentonit- oder Montmorillonit-Typ oder Aluminiumsilikate vom Zeolith-, Mordenit- oder Erionit-Typ eingesetzt werden.

3. Verfahren nach Anspruch 1 oder 2, wobei das TAA, gegebenenfalls in einem inerten organischen Lösungsmittel gelöst, bei Temperaturen von 20 bis 150°C mit überschüssigem Ammoniak umgesetzt wird.

4. Verfahren nach Anspruch 1 oder 2, wobei das TAA, gegebenenfalls in einem inerten organischen Lösungsmittel gelöst, bei Drücken von 15 bis 500 bar mit überschüssigem Ammoniak umgesetzt wird.

5. Verfahren nach einem der Ansprüche 1, 3 oder 4, wobei die Umsetzung des TAA mit dem Ammoniak in einem Reaktor durchgeführt wird, der die Katalysatoren a) und b) enthält.

## Claims

1. A process for preparing 4-amino-2,2,6,6-tetramethylpiperidine (TAD) of the formula **I** 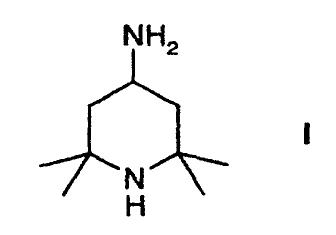 wherein, in a first step, 2,2,6,6-tetramethyl-4-piperidinone (TAA) of the formula **II** 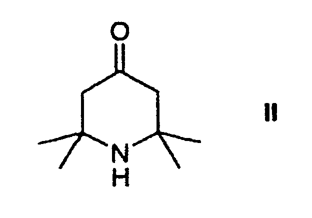 is reacted with ammonia in the presence of a) solid acidic catalysts which are virtually insoluble in the reaction mixture, to give the TAA imine of the formula **III** 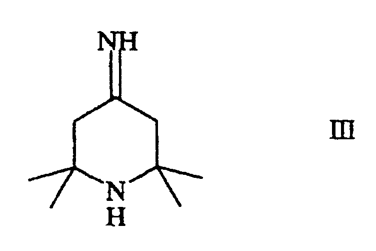 and, in a second step, the TAA imine is hydrogenated in the presence of a hydrogenation catalyst b),
wherein aluminum hydrosilicates of the bentenite or montmorillonite type or aluminum silicates of the zeolite, mordenite or erionite type are employed as catalyst a).

2. A process for preparing 2,2,6,6-tetramethyl-4-piperidinimine (TAA imine) of the formula **III** 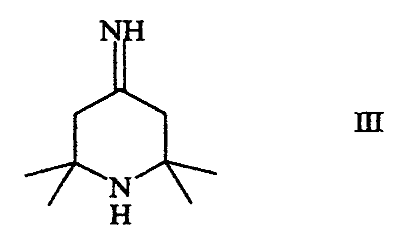 wherein 2,2,6,6-tetramethyl-4-piperidinone (TAA) of the formula **II** 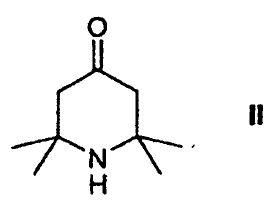 is reacted with ammonia in the presence of a) solid acidic catalysts which are virtually insoluble in the reaction mixture,
wherein aluminum hydrosilicates of the bentenite or montmorillonite type or aluminum silicates of the zeolite, mordenite or erionite type are employed as catalyst a).

3. A process as claimed in claim 1 or 2, wherein the TAA is, where appropriate dissolved in an organic solvent, reacted with excess ammonia at from 20 to 150°C.

4. A process as claimed in claim 1 or 2, wherein the TAA is, where appropriate dissolved in an organic solvent, reacted with excess ammonia under pressures of from 15 to 500 bar.

5. A process as claimed in any of claims 1, 3 or 4, wherein the reaction of TAA with the ammonia is carried out in a reactor which contains catalysts a) and b).

## Revendications

1. Procédé de préparation de 4-amino-2,2,6,6,-tétraméthylpipéridine (TAD) de formule I, 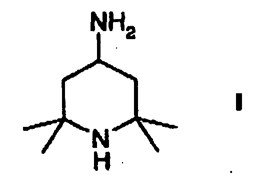 dans lequel, dans une première étape, on fait réagir de la 2,2,6,6,-tétraméthylpipéridin-4-one (TAA) de la formule II 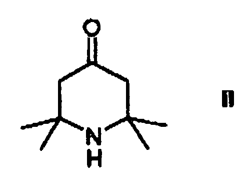 avec de l'ammoniac en présence de a) catalyseurs acides solides, qui sont pratiquement insolubles dans le mélange réactionnel, pour obtenir de la TAA-imine de formule III 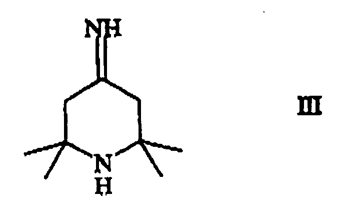 et
dans une deuxième étape, la TAA-imine est hydrogénée en présence d'un catalyseur d'hydrogénation b),
**caractérisé en ce que** l'on met en oeuvre en tant que catalyseur a) des hydrosilicates d'aluminium de type bentonite ou montmorillonite ou des silicates d'aluminium de type zéolithe, mordénite ou érionite.

2. Procédé de préparation de 2,2,6,6,-tétraméthylpipéridin-4-imine (TAA-imine) de formule III, 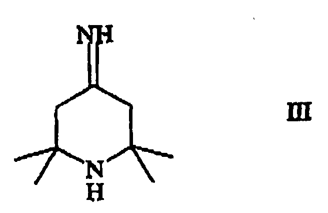 dans lequel on fait réagir de la 2,2,6,6,-tétraméthylpipéridin-4-one de formule II 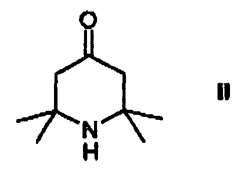 avec de l'ammoniac en présence de a) catalyseurs acides solides, qui sont pratiquement insolubles dans le mélange réactionnel, **caractérisé en ce que** l'on met en oeuvre en tant que catalyseur a) des hydrosilicates d'aluminium de type bentonite ou montmorillonite ou des silicates d'aluminium de type zéolithe, mordénite ou érionite.

3. Procédé selon la revendication 1 ou 2, dans lequel la TAA, éventuellement dissoute dans un solvant organique inerte, est mise en réaction à des températures de 20 à 150°C avec un excès d'ammoniac.

4. Procédé selon la revendication 1 ou 2, dans lequel la TAA, éventuellement dissoute dans un solvant organique inerte, est mise en réaction à des pressions de 15 à 500 bar avec un excès d'ammoniac.

5. Procédé selon l'une des revendications 1, 3 ou 4, dans lequel la réaction de la TAA avec l'ammoniac est entreprise dans un réacteur contenant les catalyseurs a) et b).
